# EUROPEAN PATENT APPLICATION

(11) **EP 2 963 420 A1**
(43) Date of publication of application: **06.01.2016**
(21) Application number: 14757636.7
(22) Date of filing: 20.02.2014
(51) Int. Cl.: G01N 33/574

(54) **BREAST-CANCER DETERMINATION METHOD**

(30) Priority: 26.02.2013 JP 2013035521
(71) Applicant: Wako Pure Chemical Industries, Ltd., Osaka 540-8605 (JP); Osaka University, Suita-shi, Osaka 565-0871 (JP)
(72) Inventor: MATSUURA, Shuji, Suita-shi Osaka 565-0871 (JP); MATSUURA, Nariaki, Suita-shi Osaka 565-0871 (JP); NAKAJIMA, Tomoyuki, Suita-shi Osaka 565-0871 (JP); KURONO, Sadamu, Osaka 559-0012 (JP)
(74) Representative: Winter, Brandl, Fürniss, Hübner, Röss, Kaiser, Polte - Partnerschaft mbB
(86) International application number: PCT/JP2014/053991
(87) International publication number: WO 2014/132869

(57) **Abstract**

The present invention provides a breast cancer marker selected from the group consisting of fucosylated hornerin, fucosylated Zn-α-2-glycoprotein, fucosylated Ig γ-1 chain C region, and fucosylated desmoplakin; a method for determining breast cancer comprising detecting the breast cancer marker in a sample, and determining on the basis of the results of the results; and a kit to be used for the determination.

## Description

### Technical Field

The present invention relates to a novel breast cancer marker, and a method for determining (diagnosing and testing) breast cancer comprising detecting the breast cancer marker and determining on the basis of the results of the detection, and a kit to be used for the determination.

### Background

At present, as a method for diagnosing breast cancer, the diagnosis on the basis of detecting a breast cancer marker in a sample such as body fluid or blood, or the diagnosis by mammography has been carried out.

In the method for diagnosing breast cancer by detecting a breast cancer marker, using a Carcinoembryonic antigen (CEA) as a marker of breast cancer, the amount of the marker in a sample is measured by using a diagnostic kit etc., and the breast cancer is diagnosed on the basis of the results of the measurement. However, since accuracy of indicating breast cancer specificity is poor, this method has not been established as a definitive diagnosis of breast cancer for practical use.

In addition, a method for identifying the patient having breast cancer or breast pre-cancer by detecting the components in the lactiferous duct fluid using the lactiferous from the subject duct fluid as a sample is disclosed in JP-A-2002-131322 (Patent Literature 1). However, although the literature describes many marker candidates including extracellular matrix protein or apolipoprotein, it lists only the marker candidates, and the analysis of component in the lactiferous duct fluid of the breast cancer patients was not carried out. Therefore, from the disclosure of Patent Literature 1, a large number of markers listed therein are unknown whether these are really useful as a breast cancer marker.

In addition, in JP-A-2008-502891 (Patent Literature 2), it has been described that a piece of tissue of breast cancer patient was analyzed by LC-ESI-MS/MS (liquid chromatography-electrospray ionization-tandem mass spectrometry), and PDX1 (peroxiredoxin 1) was selected as a candidate of breast cancer marker, and that the PXD1 was detected in the serum from breast cancer patients. In addition, there is a description suggesting that the breast cancer may be diagnosed by combinational use of PDX1 with other markers. However, the evidence indicating that these markers are detected in breast cancer patient specifically. For example, the measurement results of these markers in a sample from non-breast cancer subjects etc. is not disclosed. Therefore, PDX1 was not been confirmed whether it is useful as a marker for breast cancer, and whether the combination with other markers can be used in the diagnosis of breast cancer.

In addition, in the diagnostic method of breast cancer using mammography, mental and physical distress received by medical examinee of the test (subject) is not small. In addition, because imaging of young people with high breast density is difficult, the method has also not been accepted as a breast cancer diagnosis technology at a high level of accuracy.

Beyond that, the investigations on the search for biomarkers (proteins/peptides, lipids, sugar chains, etc.) are actively carried out in recent years, and studies on the comprehensive analyses of proteomics etc. using a variety of body fluids particularly in connection with the disease have been carried out actively. By these analyses, a highly sensitive and accurate analysis of trace sample obtained from a living organism can be carried out. In this regards, studies on the breast cancer markers conducted using proteomics is introduced by F. Mannello et al. in Expert Rev. Proteomics 6, 43-60 (2009) (Non-Patent Literature 1). However, as for the breast cancer marker, the cancer marker with high accuracy in breast cancer diagnosis has not been found yet.

### Citation List

### Patent Literature

Patent Literature 1: JP-A-2002-131322;
Patent Literature 2: JP-A-2008-502891.

### Non-Patent Literature

Non-Patent Literature 1: F. Mannello et al. Expert Rev. Proteomics 6, 43-60 (2009).

### Summary of the Invention

### Technical Problem

Because there are problems as described above in the conventional diagnosis method for breast cancer, screening rate of breast cancer has not been improved, and the diagnosis of breast cancer and early-stage breast cancer is delayed, and as a result, this has caused determent on the reduction of prevalence rate of breast cancer and mortality rate of breast cancer, and has become a major social problem. In order to solve these problems, development of a new breast cancer diagnostic technique which is minimally invasive and uses highly accurate breast cancer marker as an indicator of breast cancer diagnosis has been awaited.

An object of the present invention is to provide a novel breast cancer marker useful for detecting breast cancer, and a novel method for determining breast cancer with a high accuracy by detecting the breast cancer marker.

### Solution to Problem

The present invention was made for the purpose of solving the problems as described above, and made up of the following constituents,
(1) A breast cancer marker selected from the group consisting of fucosylated hornerin, fucosylated Zn-α-2-glycoprotein, fucosylated Ig γ-1 chain C region, and fucosylated desmoplakin.
(2) A method for determining breast cancer, comprising:
   detecting one or more breast cancer markers selected from the group consisting of fucosylated hornerin, fucosylated Zn-α-2-glycoprotein, fucosylated Ig γ-1 chain C region, and fucosylated desmoplakin in a sample, and
   determining on the basis of the results thereof.
(3) A kit for detecting breast cancer marker for determining breast cancer comprising a reagent for detecting the breast cancer marker selected from the group consisting of fucosylated hornerin, fucosylated Zn-α-2-glycoprotein, fucosylated Ig γ-1 chain C region, and fucosylated desmoplakin.

It has been known that, when cells become cancerous, a mutation of sugar chain structure such as addition of fucose (fucosylation) on the sugar chain of glycoprotein in the cell is observed.

In the course of intensive investigation aiming to establish a breast cancer marker with high accuracy in diagnosis of breast cancer which solves the above described problems and to establish a method for determining breast cancer using the same, the present inventors separated the glycoproteins in which sugar chain have been fucosylated by lectin affinity liquid chromatography (hereinafter, "liquid chromatography" is abbreviated as "LC") using the nipple discharge (ND) as a sample, from which the information on the mammary duct of onset site of breast cancer can be obtained directly. Then the components in the nipple discharge containing a large number of components were analyzed by applying proteomics with the use of nano LC-ESI-MS/MS of the most recent two-dimensional liquid chromatography (LC) / mass spectrometry (MS) system. And, using the nipple discharge derived from breast cancer patient and the nipple discharge derived from non-breast cancer subject as a sample, and by comparing the results of the nano LC-ESI-MS/MS, a new marker which is estimated to be promising as a breast cancer marker was selected. Then, the present inventors have found that when these markers are used as an indicator, it is possible to determine the breast cancer with good accuracy, and have thus completed the present invention.

### Advantageous Effects of Invention

Determination method using the breast cancer marker(s) of the present invention allows the determination (diagnosis, test) of breast cancer with higher accuracy. In addition, the breast cancer marker(s) of the present invention is found, for example, in the nipple discharge, and therefore, it is possible to determine (diagnose, test) the breast cancer non-invasively and easily.

### Brief Description of Drawings

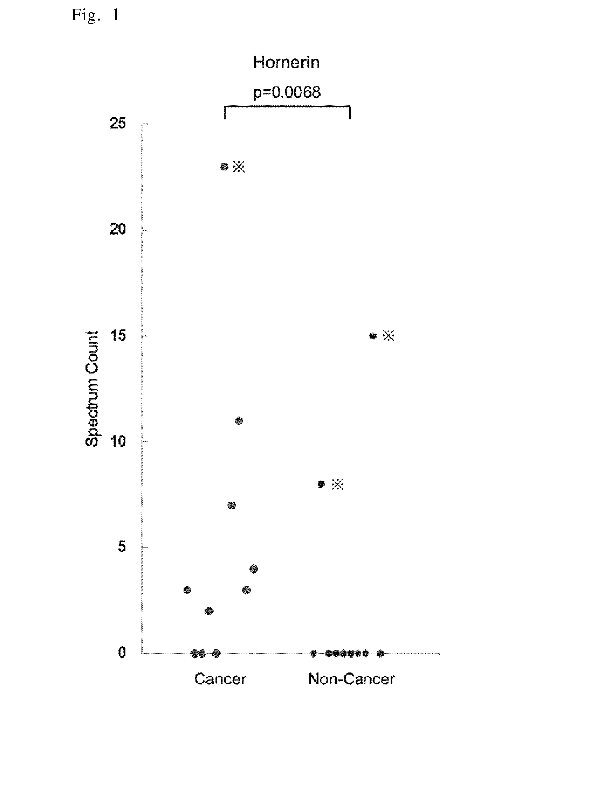

Fig. 1 shows the results of statistical analysis of the measurement results of fucosylated hornerin on the basis of the detection results of each protein component in a sample derived from breast cancer patients and non-breast cancer subjects, which were obtained in Example 1. In the figure, 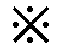 indicates upper outliers in the statistical analysis.

### Description of Embodiments

The breast cancer marker of the present invention includes a breast cancer marker selected from the group consisting of fucosylated hornerin, fucosylated Zn-α-2-glycoprotein, fucosylated Ig γ-1 chain C region, and fucosylated desmoplakin, which have a fucosylated sugar chain that is modified with a fucose, (hereinafter, these are sometimes referred to as "breast cancer marker(s) of the present invention", collectively).

Modification number of fucose, modification site, and structure of sugar chain, etc. in the fucosylated sugar chain are not particularly limited. In addition, the fucose pertaining to the fucosylation may be either L-fucose or D-fucose. Binding mode in fucosylation is also not limited, and for example, includes α-1,2 bond, α-1,3 bond, α-1,4 bond, or α-1,6 bond etc.

These proteins themselves are already known, but the usefulness of the fucosylated glycoprotein whose sugar chain is modified with fucose as an indicator (breast cancer marker) for determining breast cancer was revealed by the present inventors for the first time.

The method for obtaining the data for carrying out determination of breast cancer of the present invention is "A method for obtaining a data for detecting breast cancer, comprising detecting one or more breast cancer markers selected from the group consisting of fucosylated hornerin, fucosylated Zn-α-2-glycoprotein, fucosylated Ig γ-1 chain C region, and fucosylated desmoplakin in a sample".

The method for detecting each breast cancer marker of the present invention to be carried out for this purpose includes the conventional methods of detecting and/or measuring each component of the breast cancer marker of the present invention. Reagents for detecting and/or measuring the breast cancer marker to be used therefor may be any reagent to be used in the conventional methods for detecting and/or measuring each component of the breast cancer marker of the present invention.

An example of the method for detecting a breast cancer marker of the present invention includes the method according to the immunological measurement method well-known per se using, for example, a substance having an affinity for the breast cancer marker of the present invention (for example, antibodies, lectins, proteins, various receptors, various ligands, etc.), such as so-called enzyme immunoassay (EIA), radioimmunoassay (RIA), enzyme-linked immunosorbent assay (ELISA), fluorescence immunoassay (FIA), a measurement method by simple immunochromatography, in addition to these methods, includes the methods in combination of these methods with high-performance liquid chromatography (HPLC), electrophoresis, capillary electrophoresis, capillary chip electrophoresis method, etc. The principle of the measurement includes, for example, the sandwich method, a competitive method, double antibody method, etc., but not limited thereto.

In addition, the breast cancer marker of the present invention may be detected, for example, by a measurement method in accordance with immuno-agglutination method such as nephelometric immunoassay, turbidimetric immunoassay. These detection methods may also be carried out according to a method well-known per se.

An antibody to be used for detecting breast cancer marker of the present invention may be any antibodies which recognize fucosylated hornerin, fucosylated Zn-α-2-glycoprotein, fucosylated Ig γ-1 chain C region, or fucosylated desmoplakin, specifically. In addition, it may be, for example, either a polyclonal antibody or a monoclonal antibody as long as it has such property, and it is optional to use them alone or in appropriate combination, etc. In addition, these antibodies are digested, if necessary, with an enzyme such as pepsin or papain, and may be used in the form of F(ab')₂, Fab', or Fab. Further, a commercially available antibody against the breast cancer marker of the present invention can also be used.

In addition, the breast cancer marker of the present invention can also be detected by a conventional detection method. That is, the sample to be tested is subjected to an affinity LC method, a lectin electrophoresis method etc., using a lectin having an affinity to the fucose residue as described later, and after separating glycoproteins having fucosylated sugar chain in the sample to be tested, hornerin, Zn-α-2-glycoprotein, Ig γ-1 chain C region, or desmoplakin in a sample to be tested is detected by a conventional method.

In this regard, when an anbtibody which recognizes fucosylated hornerin, fucosylated Zn-α-2-glycoprotein, fucosylated Ig γ-1 chain C region, or fucosylated desmoplakin specifically is used, the breast cancer marker of the present invention can be detected more simply and easily without going through plural processes such as a step of separating fucosylated proteins and a step of detecting the glycoproteins.

The reagent and its concentration at the time of detection, and the measurement conditions etc. (reaction temperature, reaction time, pH at the reaction, measuring wavelength, measuring devices, etc.) on the occasion of carrying out the detection, which are used to detect the above-described breast cancer marker of the present invention, may all be set in accordance with the measurement procedure of the above described immunological assay well-known per se, and automatic analyzer or spectrophotometric system etc., which are usually used in this field, can also be used without exception.

In these reagents to be used for detection of breast cancer marker of the present invention described above, the reagents which are commonly used in this field, for example, buffering agents, reaction accelerators, sugars, proteins, salts, stabilizers such as surfactant and preservatives, and which do not inhibit the stability of coexisting reagents etc., and do not inhibit the reaction of the breast cancer marker of the present invention with the antibodies etc. against the breast cancer marker, may be included. In addition, the concentration thereof may also be selected appropriately from the range of concentrations commonly used in this field.

In addition, the breast cancer marker of the present invention may be detected in combination of two or more kinds. The combination is not particularly limited, and two or more kinds from a breast cancer marker of the present invention may be selected optionally.

Further, the combination may be any combination of one or more kinds of breast cancer markers of the present invention with one or more kinds of breast cancer markers other than the breast cancer markers of the present invention.

It should be noted that, the detection of breast cancer marker of the present invention may be carried out by a measurement system, not being limited to the manual means, but using an automatic analyzer. The combination, etc. of the reagents etc. in the case where the measurement is carried out by using the manual means or an automatic analyzer is not particularly limited, and may be used by selecting appropriately the combination of reagents assumed to be the best according to the environment of the automatic analyzer and its model to be applied or taking other factors into consideration.

Further, the breast cancer marker of the present invention may be detected by carrying out comprehensive analysis of protein by nano LC-ESI-MS/MS, after separation and fractionation of fucosylated glycoprotein that the sugar chain of the detection target is fucosylated by carrying out the so-called affinity LC using lectin column. The nano LC-ESI-MS/MS is a method that a nano-liquid chromatography is employed as a separation means, and a solution is sent at a low flow rate of several hundred nL/min, and after separating the components in a sample comprising a large number of components by the liquid chromatography, mass spectrometry by electrospray ionization method online is carried out, and then, for the mass components identified thereby, additional mass spectrometry using collision-induced dissociation is carried out once again. In this analytical method, after the mass analysis is carried out once and only specified mass component is subjected to mass spectrometry again and the obtained fragment pattern is further analyzed, therefore, a more detailed analysis can be carried out.

In the present invention, as the lectin to be used for affinity LC using the lectin column, for example, the lectins having an affinity for fucose residue, such as Aleuria aurantia lectin (Aleuria aurantia lectin, AAL) and lentil lectin (Lens culinaris agglutinin, LCA), are known. Since AAL and LCA are known to have an affinity for the fucose residue, it is possible to separate the fucosylated glycoproteins by carrying out an affinity LC of AAL or LCA. Lectin-affinity LC may be carried out by a conventional method under such condition that allows fractional isolation of the fucosylated glycoproteins. For example, a solution of mobile phase is sent at a flow rate of a few hundred µL/min, and after fucosylated glycoprotein is adsorbed onto the column using Bis-Tris aqueous solution of pH 6, etc., the fucosylated glycoproteins adsorbed onto the column are detached from the column using 20 mM aqueous L-fucose solution, the protein fractions detected by UV-absorption method using a ultraviolet light of 280 nm wavelength may be fractionated.

It should be noted that, AAL is a lectin which forms a dimer composed of two subunits having a molecular weight of about 36 kDa, and has a binding specificity for a fucose linked to N-acetylglucosamine (GlcNAc) through α-1,6 bond and a fucose linked to GlcNAc through α-1,3 bond.

Subsequently, the fucosylated glycoprotein in a fraction fractionated is digested with an enzyme, such as trypsin, and then introduced into nano LC-ESI-MS/MS.

When the breast cancer marker of the present invention is detected by nano LC-ESI-MS/MS, for example, the LC part of the above-described nano LC-ESI-MS/MS may be detected by carrying out comprehensive analysis of protein by two-dimensional nano LC-ESI-MS/MS using two separation systems with two different pHs.

A specific example of the method for detecting breast cancer marker of the present invention by two-dimensional nano LC-ESI-MS/MS is as follows.

In the liquid chromatograph of the first dimension, for example, a reverse phase column is used, and a basic solution (pH 9 to 10) such as about 20 mM aqueous ammonium formate solution or about 70 mM aqueous triethylamine solution, and acetonitrile is used as mobile phases, and the solutions are sent at a low flow rate like a few µL/min (preferably, 5 to 500 µL/min), and the components in the sample are separated, and fractionated into about dozens fractions, for example, at fixed time intervals from 1 to 10 minutes intervals. Then, after evaporating the solvent in each fraction fractionated, 0.1% aqueous trifluoroacetic acid solution for each fraction was added to dissolve the separated components.

Then, the whole volume of each fractionated solutions obtained is subjected to the liquid chromatography of the second dimension respectively, and separated in the following way. That is, a whole volume of solution of the above-described each fraction is applied to the reverse-phase chromatography column, respectively, and using as mobile phases an acidic solution (pH 2 to 3) such as 0.1% aqueous formic acid solution and acetonitrile/0.1% formic acid, the solutions are sent at a low flow rate like several ten to one hundred nL/min (preferably, 50 to 500 nL/min), and a large number of components in each fraction are separated, and ESI-MS/MS is carried out online.

Since the analysis carried out using two-dimensional nano LC-ESI-MS/MS provides more improved resolution than that carried out using one-dimensional nano LC-ESI-MS/MS, more comprehensive analysis of proteins becomes possible. By measuring with using the internal standard substance etc., the quantitative analysis is also possible.

The method for determining breast cancer of the present invention is "a method for determining breast cancer comprising detecting a breast cancer marker of the present invention and determining on the basis of the result thereof". That is, by the method described above, the breast cancer marker of the present invention is detected, and based on the results of the detection, the data (for example, information on the presence or absence, the extent of the increase in the concentration and the amount of the breast cancer marker of the present invention) for determining breast cancer using the breast cancer marker of the present invention as an indicator is obtained. Using the data obtained, for example, by the following method, determination (diagnosis and test) of breast cancer is carried out.

That is, for example, detection of the breast cancer marker of the present invention in a sample derived from a subject is carried out, and from the test result stating that the breast cancer marker is detected, it is determined that the subject might be at risk of breast cancer, or the subject is in high risk of breast cancer. In addition, in the case that the breast cancer marker has not been detected, it is determined that there is no risk of breast cancer in the subject, or the risk of breast cancer is low, etc.

As another method, at first, the amount of breast cancer marker of the present invention contained in a sample is determined by the above described method. Then the obtained result is compared with the result obtained by the same manner using a sample derived from non-breast cancer subject as a separate sample in advance, and from the test result stating that the amount of the breast cancer marker contained in a sample derived from the subject is greater than that contained in a sample derived from non-breast cancer subject, it is possible to determine that the subject might be at risk of breast cancer, or the subject is in high risk of breast cancer. In addition, from the test result stating that a significant difference is not observed between the amount of the breast cancer marker contained in the sample derived from the subject and the amount contained in the sample derived from non-breast cancer subject, it is possible to determine that there is no risk of breast cancer in the subject, or the risk of breast cancer is low.

In addition, a reference value is set in advance, and from the test results stating that the amount of breast cancer marker of the present invention from a subject is more than the reference value of the breast cancer marker, it is possible to determine that there is at risk of breast cancer in the subject, or the risk of breast cancer is high in the subject, etc. In addition, setting plural judgment criteria corresponding to the amount of the breast cancer marker or the quantitative range thereof [for example, (1) no risk of breast cancer, (2) low risk of breast cancer, (3) sign of breast cancer, (4) high risk of breast cancer, etc.], it is also possible to determine whether the test result corresponds to which judgment criteria.

Furthermore, in the same subject, by comparing the amount of the breast cancer marker of the present invention in a sample derived from the subject measured at some time point with the amount of the breast cancer marker at different time points, and by evaluating the presence or absence of increase or decrease, and/or the degree of increase or decrease of the breast cancer marker, diagnosis of progression or malignancy of breast cancer, or prognosis after surgery is possible. That is, from the test results stating that an increase in the amount of the breast cancer marker was observed, a determination that the pathological condition has progressed to breast cancer (or malignancy of breast cancer has increased), or a sign of pathological progression to breast cancer was observed (or a sign of increase in malignancy of breast cancer was observed), can be made. In addition, from the test results stating that an decrease in the amount of the breast cancer marker in a sample derived from the subject has been observed, a determination that the pathological condition of breast cancer was improved, or a sign of improvement of pathological condition was observed, can be made. In addition, from the test result stating that variation in the amount of the breast cancer marker is not observed, a determination that there is no change in the pathological condition can be made.

Further, a method in which two or more breast cancer markers of the present invention are detected and measured, and determination of breast cancer is made on the basis of the results, is also included. For example, by detecting breast cancer marker of the present invention alone, or two or more kinds in combination, the presence pattern of breast cancer markers of the present invention characteristic of the breast cancer patients can be obtained. Therefore, by detecting and identifying breast cancer markers of the present invention from a sample of the nipple discharge, etc. from the subject, and by analyzing the presence pattern of these plural breast cancer markers of the present invention, the estimation whether or not the subject is developing breast cancer is possible.

The combination of two or more of breast cancer markers may be selected optionally from the group consisting of breast cancer markers of the present invention.

In addition, using any combination of one or more of the breast cancer markers of the present invention and one or more of breast cancer markers other than the breast cancer markers of the present invention, detection of the present invention may also be carried out. In this case, respective breast cancer markers are detected and measured, and determination of breast cancer may be carried out in the same manner as described above.

The sample to be used for detecting breast cancer marker of the present invention includes, nipple discharge of a subject, or a sample derived from a living organism such as blood (whole blood), serum, plasma, urine and lymph, but not limited thereto.

The nipple discharge includes the nipple discharge secreted naturally from breast of one or both sides of the subject, secretory fluid exuded by massage of the breast and nipple aspirate fluid, etc., but it is not limited thereto.

If the nipple discharge is used as a sample, the sample can be taken from the subject non-invasively, and it is excellent in the terms that burden on the patient is much less.

"A kit for detecting a breast cancer marker for determining breast cancer" of the present invention is the one which comprises the reagents for detecting breast cancer marker of the present invention as a constituent requirement.

Preferred embodiment and specific example of "the reagents for detecting breast cancer marker of the present invention" and the constituent requirement thereof is as described above in the description on the method for obtaining a data for determining a breast cancer of the present invention. In addition, a preferred embodiment of the concentration, etc. of these reagents may be selected appropriately from the range of concentrations usually used in this field.

For example, an example of the reagents for detecting the breast cancer marker of the present invention includes the one which contains a substance having affinity for the breast cancer marker of the present invention. In addition, an example of the substance having affinity for the breast cancer marker of the present invention include, for example, antibody, lectin etc., and specific examples thereof is as described above in the description on the method for obtaining the data for determining a breast cancer.

The kit may be either the one which comprises reagents or a kit for detecting one kind of breast cancer marker as the constituent requirements, or the one which comprises a combination of plural reagents or kits for detecting plural kinds of breast cancer marker.

In addition, in the reagent contained in these kits, there may be included reagents which are commonly used in this field, for example, buffering agents, reaction accelerators, sugars, proteins, salts, stabilizers, such as surfactant, and preservatives, and which do not inhibit the stability of coexisting reagents etc., and do not inhibit the reaction of the breast cancer marker of the present invention and an antibody (or lectin, etc.). In addition, the concentration thereof may also be selected appropriately from the range of concentrations commonly used in this field.

Further, it may be a kit which is made up by combining the standard of breast cancer marker for preparing a standard curve to be used on the occasion of detecting the breast cancer marker. For the standards, the commercially available standards or those produced according to known methods, may also be used.

Furthermore, in the kit of the present invention, a manual etc. for use in the method for obtaining data for carrying out determination of breast cancer of the present invention or the method for determining breast cancer may be included. The "manual" means the instruction manual, the package insert, or brochure (leaflet), etc. of the kit, in which features, principles, operating procedures, and determination procedures, etc. of the method is described substantially in sentence or by figures and tables.

Hereinafter, the present invention is explained specifically based on examples, but the scope of the invention should not be limited thereto.

### Examples

### Example 1: Selection of breast cancer marker

### (1) Pre-treatment of sample to be introduced into lectin affinity LC using AAL (Aleuria aurantia Lectin)

Respective nipple discharge (a few µL) taken from breast cancer patients (10 persons) was added to 100 µL of sample stock solution (an aqueous solution containing 50 mM ammonium hydrogencarbonate and 0.02% sodium azide), and centrifuged at 18,000 x g, for 5 minutes at 4°C. After separation of supernatant, 5 µL aliquot of the supernatant was diluted 20-fold with AAL Adsorption buffer (GALAB Technologies, Geesthacht, Germany), and 100 µL of the obtained sample was subjected to the AAL affinity LC by the following manner.

Separately, the nipple discharge collected from medical examinee (subject) (10 persons) who appealed that secretory fluid was secreted from a nipple, as an indefinite complaint, and who were diagnosed as non-breast cancer, were treated in the same manner as described above.

### (2) Measurement of lectin-affinity LC using AAL

The sample obtained in the above-described (1) was injected into HPLC instrument (1200 series, Agilent Technologies, Morges, Switzerland) equipped with Affisep-AAL Affinity column (4 mm i.d. x 5 cm) (GALAB Technologies, Geesthacht, Germany). As the mobile phase A and B, AAL Adsorption buffer and AAL Elution buffer (GALAB Technologies, Geesthacht, Germany) were used, respectively. For the purpose of separating AAL nonbinding protein and AAL binding protein in a sample, separation was carried out by a flow rate at 250 µL/min and by stepwise for 50 min, and AAL nonbinding fraction (0 min to 6 min) and AAL binding fraction (22 min to 38 min) were isolated, respectively.

Each condition of AAL affinity LC measurement was as follows:
HPLC column: Affisep-AAL Affinity column, 4 mm i.d. x 5 cm (produced by GALAB Technologies);
Flow rate: 250 µL/min;
Solvent A: AAL Adsorption buffer (produced by GALAB Technologies);
Solvent B: AAL Elution buffer (produced by GALAB Technologies);
Elution: 0 min → 20 min: 0% Solvent B,
   20 min → 35 min: 100% Solvent B,
   35 min → 50 min: Equilibration with 0% Solvent B;
Column temperature: 20°C;
Injection volume: 100 µL.

### (3) Pretreatment of the sample to be introduced into the First dimensional micro LC of two-dimensional nano LC

The AAL binding fraction fractionated by AAL affinity LC in the above-described (2) was subjected to ultrafiltration (7,500 x g, for 40 min) using Amicon Ultra 3K (Millipore Corporation, Billerica, MA, USA), and further, after carrying out solvent substitution (7,500 x g, for 40 min) by adding 1 mL of 50 mM ammonium hydrogencarbonate, the protein concentration was determined by ultraviolet absorption method using ultraviolet light of 280 nm wavelength. To an approximately 100 µL of the solvent substituted sample, 1 µL of 45 mM dithiothreitol solution was added and reduced, then trifluoroethanol was added to denature the protein so as to provide 50%, and then the reaction solution was heated at 60°C for 1 hour. The reaction solution was returned to room temperature after heating, and was subjected to alkylation by adding 1 µL of 100 mM iodoacetamide solution at room temperature under a dark environment for 1 hour. Then the reaction solution was adjusted by adding 50 mM aqueous ammonium hydrogencarbonate solution so that the percentage of trifluoroethanol becomes 5%. Subsequently, a 100 ng/µL trypsin solution (mass spectrometry grade, Promega Corporation, Madison, WI, USA) adjusted so as to provide the amount of trypsin is 1/20 weight of the protein weight was added, and incubated at 37°C overnight. The obtained trypsin digestion product was adjusted to be about 250 fmol/µL in bovine serum albumin equivalent, and two-dimensional nano LC-ESI-MS/MS measurement was carried out by the following way.

### (4) Two-dimensional nano LC-ESI-MS/MS measurement

A 40 µL aliquot of the trypsin digestion product obtained in the above-described (3) was separated using Acclaim PA2, 300 µm i.d. x 15 cm reverse-phase column (Thermo Fisher Scientific Inc. (Dionex), Waltham, MA, USA) which was equipped in a two-dimensional Nano HPLC system (UltiMate 3000; Thermo Fisher Scientific Inc. (Dionex), Waltham, MA, USA) by gradient elution using solvent A : 20 mM aqueous ammonium formate solution, pH 10.0 and solvent B : acetonitrile for 75 minutes (12 to 95% of solvent B), and 26 fractions were obtained. The solvent in those fractions was evaporated by heating at 70°C for 4 hours. Then, 30 µL of 0.1% aqueous trifluoroacetic acid solution was added to each fraction to dissolve the separated components. Subsequently, using a L-column Micro L2-C18, 75 µm i.d. x 15 cm reverse phase column (produced by Chemicals Evaluation and Research Institute, Japan), each of the whole volume of each fraction solution obtained was separated by gradient elution using solvent A : 0.1% aqueous formic acid solution and solvent B : acetonitrile/0.1% formic acid for 45 minutes (2 to 95% of solvent B), and analyzed sequentially online using data-dependent scan by ESI-ion trap mass spectrometer (HCTultra; Bruker Daltonik GmbH, Bremen, Germany) equipped with nano-ESI ion source. For each sample, run was performed twice.

Each condition of the two-dimensional nano LC-ESI-MS/MS analysis was as follows:

### i) LC condition

### (First-dimensional)

HPLC column: Acclaim PA2, 300 µm i.d. x 15 cm reverse phase column (produced by Thermo Fisher Scientific Inc. (Dionex));
Flow rate: 6.0 µL/min;
Solvent A: 20 mM aqueous ammonium formate solution, pH 10.0;
Solvent B: Acetonitrile;
Elution: 0 min → 19 min: Linear gradient from 12% to 20% solvent B,
19 min → 29 min: Linear gradient until 48% solvent B,
29 min → 34 min: Washing with 95% solvent B,
34 minutes → 75 minutes: Equilibration with 2% solvent B;

(It should be noted that, since the concentrations of solvent B was raised immediately after the start of elution, the concentration of solvent B in the eluent has become 12% in almost 0 minutes after the start of elution.)
Column temperature: 35°C;
Injection volume: 40 µL;
Number of fractions: 26.

### (Second dimension)

HPLC column: L-column Micro L2-C18, 75 µm i.d. x 15 cm reverse phase column (produced by Chemicals Evaluation and Research Institute, Japan);
Flow rate: 300 nL/min;
Solvent A: 0.1% aqueous formic acid solution, pH 2.0;
Solvent B: Acetonitrile/0.1% formic acid;
Elution: 0 min → 1 min: Linear gradient until 8% solvent B,
1 min → 20 min: Linear gradient until 20% solvent B,
20 min → 25 min: Linear gradient until 35% solvent B,
25 min → 30 min: Washing with 95% solvent B,
30 min → 45 min: Equilibration with 2% solvent B;
Column temperature: Room temperature;
Injection volume: 30 µL;

### ii) ESI-MS/MS condition

Ionization method: Electrospray ionization method (ESI method);
Measured ion: Positive ion;
Spray voltage: 1200 V;
Dry gas: Nitrogen (4 L/min);
Ion source temperature: 160°C;
Scanning range: *m*/*z* 300 to 1,500 (at MS/MS measurement: *m*/*z* 50 to 2,200);
Precursor ion selection number: 4 ions/mass spectrum;
Minimum precursor ion intensity: 50,000 counts;
Precursor ion selective elimination time: 30 s;
Precursor ion elimination valence: 1.

### iii) Protein identification analysis condition

Protein search engine: Mascot (Matrix Science Ltd., London, UK);
Protein search technique: MS/MS Ion Search;
Protein databases: Swiss-Prot;
Animal species classification at protein search: Homo sapiens (human);
Post-translational modification setting at protein search: Carbamidomethyl (cysteine residue modification);
Trypsin non-cleavage site allowable number of times: 1;
Precursor ion mass tolerance: ± 0.5 Da;
Product ion mass tolerance: ± 1 Da;

Protein identification and analysis software: Scaffold (Proteome Software, Inc., Portland, OR, USA).

### (5) Results of two-dimensional nano LC-ESI-MS/MS measurement

By two-dimensional nano LC-ESI-MS/MS measurement, 82 species of protein components in samples derived from breast cancer patients, 86 species of protein components in samples derived from non-breast cancer subjects, a total of 119 species of protein components were detected. In this analysis, it should be noted that peroxiredoxin 1 described in Patent Literature 2 was not detected in both samples from breast cancer patients and from non-breast cancer subjects.

Subsequently, by the following way, proteins detected specifically from the samples from breast cancer patients were selected as breast cancer marker candidates.

### (6) Screening of breast cancer marker candidate

Based on the results of the above-described (5),
(i) Rate of number (n1) of breast cancer patients in whom each protein component was detected in a sample, among 10 persons of breast cancer patients examined (Ratio A);
(ii) Rate of number of non-breast cancer subjects (n2) in whom each protein component was detected in a sample, among 10 persons of non-breast cancer subjects examined (Ratio B); and
(iii) Ratio A / Ratio B (Ratio C),
were calculated.

For want of space, among 119 fucosylated protein components detected in the above (5), a list of fucosylated protein components whose Ratio C was 2.00 or more were shown in Table 1 below.

**Table 1**

| Detected Component (fucosylated protein) |
|---|
| Hornerin |
| Zinc-alpha-2-glycoprotein |
| Ig gamma-1 chain C region |
| Keratin, type I cytoskeletal 16 |
| Desmoplakin |
| Hemopexin |
| Plasma protease C1 inhibitor |
| Alpha-1-acid glycoprotein 2 |
| Ig lambda chain V-III region LOI |
| Catalase |
| Serotransferrin |
| Ceruloplasmin |
| Ig heavy chain V-III region GAL |
| Dermcidin |
| Junction plakoglobin |

And, the protein components etc. which were suspected to blood-derived components etc. were excluded from the list of the protein components listed in Table 1, and the protein components in which the rate of breast cancer patients from whom the protein components were detected was 30% or more (Ratio A) of the total breast cancer patients as well as the Ratio C was 2.0 or more were selected as a breast cancer marker candidate, and the Ratio A, B, and C of respective breast cancer markers were shown in Table 2.

In addition, among these breast cancer markers, as an example, statistical results of hornerin were shown in Fig. 1. In Fig. 1, the values marked by 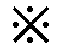 indicate upper outliers in the statistical analysis, and were excluded from the object of statistical analysis. As a result of statistical analysis, in hornerin (fucosylated hornerin), a statistically significant difference was observed between breast cancer patient and non-breast cancer subject in a spectrum count value indicating the abundance of the protein component calculated based on the peptide peaks detected in the mass spectrum (p=0.0068 in the U-test of Mann-Whitney).

In addition, with respect to fucosylated Zn-α-2-glycoprotein, fucosylated Ig γ-1 chain C region, and fucosylated desmoplakin which are other breast cancer markers of the present invention, when the same statistical analysis was carried out, statistically significant difference was observed similarly between breast cancer patients and non-breast cancer subjects.

**Table 2**

| | molecular weight | breast cancer patient | | non-breast canser sub ect | | RatioA/ ratio B |
|---|---|---|---|---|---|---|
| | | n1 | Ratio A | n2 | RatioB | Ratio C |
| fucosylated hornerin | 282kDa | 7 | 0.700 | 2 | 0.200 | 3.500 |
| fucosylated Zn-α-2-glycoprotein | 34kDa | 3 | 0.300 | 1 | 0.100 | 3.000 |
| fucosylated Ig γ-1 chain C region | 36kDa | 8 | 0.800 | 3 | 0.300 | 2.667 |
| fucosylated desmoplakin. | 332kDa | 4 | 0.400 | 2 | 0.200 | 2.000 |

Therefore, fucosylated hornerin (Hornerin), fucosylated Zn-α-2-glycoprotein (Zinc-alpha-2-glycoprotein), fucosylated Ig γ-1 chain C region (Ig gamma-1 chain C region), and fucosylated desmoplakin (Desmoplakin) as listed in Table 2 were selected as a breast cancer marker of the present invention.

As is clear from Table 2 and Fig. 1, these breast cancer markers were present in high concentrations in the nipple discharge of breast cancer patients as compared with the concentration in the nipple discharge of non-breast cancer subjects. In addition, since there is no track record of these components that was used as a breast cancer marker, these are very promising as a novel breast cancer marker.

Further, it can be said that, in the present Example, a process of concentration and separation of protein by AAL has played a very important step. Although data were not shown, for example, Zn-α-2-glycoprotein was detected in all specimens of malignant cases and benign cases. However, in the present Example, among the Zn-α-2-glycoproteins, only those having a fucosylated sugar chain were extracted by passing through the selection process by AAL, and therefore it turned out that, although the Zn-α-2-glycoprotein itself cannot be a breast cancer marker, if it is fucosylated Zn-α-2-glycoprotein, it can be utilized as a novel breast cancer marker.

In addition, it is expected that the determination of breast cancer as well as the determination of progression thereof can be carried out by detecting breast cancer marker of the present invention alone, or 2 or more kinds thereof in combination. For example, by detecting these breast cancer makers alone, or 2 or more kinds thereof in combination, the presence pattern of the breast cancer marker(s) of the present invention characteristic of breast cancer patients is obtained. Therefore, it is expected that, by detecting and identifying the breast cancer markers of the present invention from a sample of the nipple discharge, etc. of the subject, and by analyzing the presence pattern of these plural breast cancer markers, the estimation whether or not the subject has developed breast cancer will be possible.

### Industrial Applicability

The determination method using the breast cancer marker(s) of the present invention enables the determination (diagnosis, test) of breast cancer with higher accuracy.

In addition, since the breast cancer marker(s) of the present invention are found, for example, in nipple discharge, it is possible to perform determination (diagnosis, test) of breast cancer non-invasively and simply. As a result, the breast cancer diagnosis according to the present invention in which mental and physical distress is small is expected to encourage the younger generation and the young parous and multiparous women during or after lactation to perform breast cancer screening on a voluntary basis, and expected to improve the breast cancer screening rate in Japan which is lower as compared with that in the world, and to provide the economic effect due to the reduction of breast cancer mortality.

## Claims

1. A breast cancer marker selected from the group consisting of fucosylated hornerin, fucosylated Zn-α-2-glycoprotein, fucosylated Ig γ-1 chain C region, and fucosylated desmoplakin.

2. A method for obtaining a data for determining breast cancer, comprising detecting one or more breast cancer markers selected from the group consisting of fucosylated hornerin, fucosylated Zn-α-2-glycoprotein, fucosylated Ig γ-1 chain C region, and fucosylated desmoplakin in a sample.

3. The method according to claim 2, wherein the sample is nipple discharge.

4. The method according to claim 2, wherein the sample is serum, plasma, or whole blood.

5. A method for determining breast cancer, comprising:
detecting one or more breast cancer markers selected from the group consisting of fucosylated hornerin, fucosylated Zn-α-2-glycoprotein, fucosylated Ig γ-1 chain C region, and fucosylated desmoplakin in a sample, and
determining on the basis of the results thereof.

6. The method according to claim 5, wherein the sample is nipple discharge.

7. The method according to claim 5, wherein the sample is serum, plasma, or whole blood.

8. A kit for detecting a breast cancer marker for determining breast cancer comprising a reagent for detecting the breast cancer marker selected from the group consisting of fucosylated hornerin, fucosylated Zn-α-2-glycoprotein, fucosylated Ig γ-1 chain C region, and fucosylated desmoplakin.

9. The kit according to claim 8, wherein the reagents for detecting a breast cancer marker comprises a substance having an affinity for the breast cancer marker.

10. The kit according to claim 9, wherein the substance having an affinity for the breast cancer marker is a lectin or an antibody.

11. The kit according to claim 10, wherein the lectin is Aleuria aurantia lectin.

12. The kit according to claim 6, which contains a manual for use in the method described in claim 2 or 5.
